**Europäisches Patentamt**

**European Patent Office**

**Office européen des brevets**

(19)

(11) **EP 1 265 068 A1**

(12) **EUROPÄISCHE PATENTANMELDUNG**

(43) Veröffentlichungstag:
**11.12.2002 Patentblatt 2002/50**

(51) Int Cl.7: **G01N 33/22**, G01N 25/18,
G01N 25/20

(21) Anmeldenummer: **01810546.0**

(22) Anmeldetag: **05.06.2001**

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(71) Anmelder: **ABB RESEARCH LTD.
8050 Zürich (CH)**

(72) Erfinder:
• **Matter, Daniel
5200 Brugg (CH)**

• **Weisenstein, Wolfgang
5453 Remetschwil (CH)**
• **Prêtre, Philippe
5405 Baden-Dättwil (CH)**
• **Kleiner, Thomas
5442 Fislisbach (CH)**

(74) Vertreter: **ABB Patent Attorneys
c/o ABB Schweiz AG
Brown Boveri Strasse 6
5400 Baden (CH)**

(54) **Verfahren und Vorrichtung zur Bestimmung einer Änderung des Heizwertes eines Gasgemisches**

(57) In einem Verfahren zur Bestimmung einer Änderung des Heizwertes eines Gasgemisches wird die Diffusivität innerhalb des Gasgemisches bei einem konstanten, vordefinierten Massenstrom gemessen und die gemessene Diffusivität mit dem Referenzwert eines Kalibriergases mit bekanntem Heizwert verglichen. Dabei ist eine Abweichung der gemessenen Diffusivität von dem Referenzwert ein Mass für die Änderung des Heizwertes des Gasgemisches. Dies ermöglicht eine einfache und kostengünstige Bestimmung der Änderungen von Zusammensetzungen von Gasgemischen und somit von relativen Heizwertänderungen. Ein Anwendungsbereich ist die Regelung von Gasturbinen.

Fig. 1a

EP 1 265 068 A1

## Beschreibung

### Technisches Gebiet

Die Erfindung bezieht sich auf ein Verfahren und eine Vorrichtung zur Bestimmung einer Änderung des Heizwertes eines Gasgemisches gemäss Oberbegriff der Patentansprüche 1 bzw. 6.
Das Verfahren und die Vorrichtung sind insbesondere zur Online-Heizwertbestimmung bei der Regelung von Gasturbinen im Kraftwerksbereich geeignet.

### Stand der Technik

Für die Optimierung der Prozesstechnik und somit für die Regelung von Gasturbinen ist eine sehr genaue Kenntnis der Prozessparameter notwendig. Bei Gasturbinen wird in erster Näherung von einem konstanten Luftmassenstrom in eine Brennkammer ausgegangen, so dass die thermische Energie, welche für die Wellenleistung und damit für die Ausgangsleistung eines Kraftwerks verantwortlich ist, allein über die Brennstoffmenge eingestellt wird. In modernen Industriegasturbinen liegt der optimale Betriebspunkt nahe bei der mageren Löschgrenze der adiabaten Flammentemperatur, bei der die Flamme eben noch in der Lage ist, sich selber zu stabilisieren. Diese adiabate Flammentemperatur hängt unter anderem vom Heizwert des Brennstoffes ab.

Üblicherweise wird Erdgas als Brennstoff verwendet. Das vom Gaslieferanten gelieferte Erdgas unterliegt jedoch starken Schwankungen in der Qualität und somit in seinem Brennwert oder Heizwert. Diese Schwankungen werden dadurch verursacht, dass Erdgas von verschiedenen Quellen gleichzeitig geliefert wird, wobei das Mischungsverhältnis je nach Angebot stark variieren kann. Derartige Schwankungen beeinträchtigen die Funktion der Gasturbine in ihrem Wirkungsgrad, in der Stabilität der Verbrennung und auch in ihren Schadstoffemissionen. In extremen Fällen ist es sogar bereits schon zu Schäden an Anlagen gekommen.

Zur Optimierung der Prozesstechnik werden deshalb Heizwertmessverfahren verwendet, welche den Brennwert des zugeführten Brennstoffes in regelmässigen Abständen bestimmen und einem Regler übermitteln. Normalerweise werden hierfür Kalorimeter oder Gas-Chromatographen eingesetzt. Diese haben zusätzlich zu ihrem relativ hohen Preis und ihrem hohen Wartungsbedarf den Nachteil, dass sie nur eine intermitierende Messung erlauben.

Ferner offenbart DE-A-4'118'781 ein Verfahren und eine Vorrichtung zur verbrennungslosen Bestimmung des Brennwerts eines Gases. Hierfür wird in einem Massenstrommesser der Massenstrom, in einem Volumenstrommesser der Volumenstrom und in einem weiteren Messmittel die Druckdifferenz bestimmt. Daraus wird bei bekanntem Absolutdruck und bekannter Gastemperatur mit Hilfe einer Näherungsfunktion der Brennwert berechnet.

EP-A-0'469'649 offenbart eine Vorrichtung zur Bestimmung des Heizwertes mit einem Volumenflussmeter, welches den Volumenfluss anhand der Druckdifferenz zwischen laminaren Flusselementen bestimmt. In Serie mit dem Volumenflussmesser ist ein thermisches Massenflussmeter angeordnet, um den Massenfluss zu bestimmen.

Ferner ist ein Thermometer vorhanden, um die aktuelle Gastemperatur zu bestimmen, und ein Manometer, um den Absolutdruck zu messen. Anhand dieser Messwerte wird unter anderem die Druckdifferenz berechnet, woraus sich in umgekehrter Proportionalität der Heizwert ergibt.

Diese zwei Verfahren sind relativ aufwendig und die zugehörige Messvorrichtungen entsprechend teuer.

### Darstellung der Erfindung

Es ist deshalb Aufgabe der Erfindung, ein Verfahren und eine Vorrichtung zur Bestimmung einer Änderung des Heizwertes eines Gasgemisches zu schaffen, welche einfach und kostengünstig sind, jedoch trotzdem eine Online-Messung der Änderung erlauben.

Diese Aufgabe lösen ein Verfahren und eine Vorrichtung mit den Merkmalen des Patentanspruches 1 beziehungsweise 6.

Erfindungsgemäss wird ein Messsignal eines Sensors bei konstantem Durchfluss bestimmt. Die Änderung des Messsignals ist ein Mass für die Änderung des Heiz- oder Brennwertes des Gasgemisches. Dadurch lässt sich auf einfache Art und Weise eine zeitliche Änderung des Heizwertes detektieren und dieses Messsignal zur Regelung von Anlagen einsetzen.

Insbesondere ist dieses erfindungsgemässe Verfahren und die zugehörige Vorrichtung geeignet, um bei die Regelung von Gasturbinen verwendet zu werden. Sie lassen sich jedoch auch in anderen Bereichen der Gasverbrennung der chemischen und petrochemischen Industrie einsetzen, ebenso in Brennstoffzellen und zur Steuerung von heterogen-katalytischen Reaktionen.

In einer ersten bevorzugten Variante des Verfahrens wird der Massenfluss konstant gehalten. In einer zweiten bevorzugten Variante wird der Volumenfluss konstant gehalten, eine Dichte des Gasgemisches im Volumenfluss gemessen und daraus der Massenfluss bestimmt.

In einer bevorzugten Ausführungsform ist die Vorrichtung zur Bestimmung einer Änderung des Heizwertes eines Gasgemisches als Massenflussmeter ausgelegt. In einer weiteren bevorzugten Ausführungsform ist die Vorrichtung als Energieflussmeter ausgelegt.

In einer bevorzugten Ausführungsform weist die Vorrichtung einen Durchflusssensor auf, wie er in der Deutschen Patentanmeldung Nr. 199 08 664.8 und der noch unveröffentlichten Europäischen Patentanmeldung Nr. 008100511.6 beschrieben ist.

Weitere vorteilhafte Varianten des Verfahrens und vorteilhafte Ausführungsformen gehen aus den abhängigen Patentansprüchen hervor.

**Kurze Beschreibung der Zeichnungen**

Im folgenden wird der Erfindungsgegenstand anhand eines bevorzugten Ausführungsbeispiels, welches in den beiliegenden Zeichnungen dargestellt ist, erläutert. Es zeigen:

Figur 1a     eine schematische Darstellung eines Teils einer Gasleitung mit einer erfindungsgemässen Vorrichtung zur Bestimmung der Heizwertänderung eines Gases in einem Niederdrucksystem;

Figur 1b     in einem Hochdrucksystem und

Figur 2     einen Vergleich einer Abweichung von Monatsmittelwerten von Heizwerten von Erdgas und entsprechende Messwertänderungen des erfindungsgemässen Gaszählers bei konstantem Durchfluss.

**Wege zur Ausführung der Erfindung**

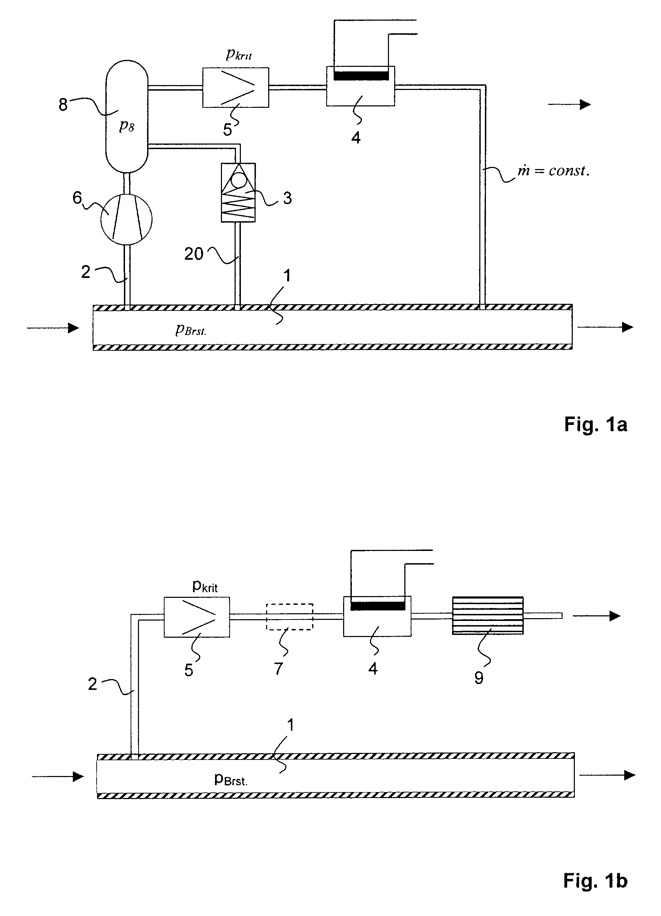

In Figur 1 ist eine Gasleitung dargestellt, welche mit einer erfindungsgemässen Vorrichtung zur Bestimmung einer Änderung des Heizwertes eines Gasgemisches versehen ist. Die Gasleitung besteht aus einem Hauptleitungsrohr 1, welches mit einer hier nicht dargestellten Anlage, beispielsweise mit einer Gasturbine verbunden ist. Durch das Hauptleitungsrohr 1 strömt normalerweise ein Gasgemisch mit zeitlich variierender Zusammensetzung, insbesondere Erdgas. Das erfindungsgemässe Verfahren und die Vorrichtung funktionieren am besten mit kohlenwasserstoffhaltigen Gasgemischen, deren Hauptbestandteile vorzugsweise Methan, Propan und Ethan sind.

Vom Hauptleitungsrohr 1 zweigt ein Messrohr 2 ab. Das Messrohr 2 weist normalerweise einen wesentlich kleineren Querschnitt auf als das Hauptleitungsrohr 1.

Es wird zwischen Niederdrucksystemen mit einem Druck im Hauptleitungsrohr bis zu 2 bar und Hochdrucksystemen mit einem Druck ab 2 bar unterschieden. Bei Niederdrucksystemen sind, wie in Figur 1a dargestellt ist, in Strömungsrichtung durch das Messrohr 2 folgende Elemente im Messrohr 2 angeordnet: ein Heizwertsensor 4, optional ein Dichtesensor 7, eine sonische Düse 5 und eine Pumpe 6. Die sonische Düse 5 dient dazu, den Massenfluss durch das Messrohr 2 konstant zu halten. Die Pumpe 6 pumpt Gas durch das Messrohr 2, vorzugsweise mit einem Durchsatz von mindestens annähernd 0.2 l/min, in ein Puffervolumen 8. Die Druckdifferenz zwischen dem Druck im Puffervolumen $p_8$ und dem Brennstoffdruck im Hauptleitungsrohr $p_{Brst.}$ ist immer wesentlich grösser als der kritische Druck der sonischen Düse 5. Dadurch ist gewährleistet, dass immer ein konstanter Massenstrom den Heizwertsensor durchströmt. Die Pumpe 6 läuft mit konstanter Leistung, der Druck im Pufferbehälter 8 wird durch ein Druckregulierventil 3 konstant gehalten indem überschüssiges Messgas durch eine Bypassleitung 20 zurück in das Hauptleitungsrohr geleitet wird. Da das Messgas beim Durchströmen des Heizwertsensors chemisch nicht verändert wird kann es nach Durchfliessen der Messstrecke zurück in das Hauptleitungsrohr 1 geleitet werden. Optional besteht auch die Möglichkeit, wie dies in Figur 1b dargestellt ist, das Messgas in einer Oxidationsvorrichtung 9 über einen Katalysator oder eine Flamme zu oxidieren und so einer Explosionsgefahr vorzubeugen. In diesem Fall endet das Messrohr an seinem dem Hauptleitungsrohr 1 fernen Ende frei und gibt somit das Gasgemisch in die Umwelt ab. Als Katalysator wird beispielsweise Paladium-Oxid (PdO eingesetzt.

Bei Hochdrucksystemen sind, wie in Figur 1b dargestellt ist, in Strömungsrichtung durch das Messrohr 2 folgende Elemente im Messrohr 2 angeordnet: eine sonische Düse 5, optional ein Dichtesensor 7 und ein Heizwertsensor 4. Eine Pumpe ist hier nur notwendig, wenn die Differenz zwischen Brennstoffdruck $p_{Brst.}$ und Atmosphärendruck nicht grösser ist wie der kritische Druck $p_{krit.}$ der sonischen Düse 5. Wird keine Pumpe zur Druckerhöhung eingesetzt, so kann das Messgas nicht in das Hauptleitungsrohr 1 zurückgeführt werden. In diesem Fall wird das Messgas in einer Oxidationsvorrichtung 9 homogen oder heterogen-katalytisch oxidiert.

Der Heizwertsensor 4 misst die Diffusivität des durchströmenden Gases. Die Diffusivität ist definiert als

$$\alpha = \frac{\kappa}{c_\rho * \rho}$$

In einem bevorzugten Ausführungsbeispiel weist der Sensor 4 mindestens zwei in Strömungsrichtung nacheinander angeordnete Temperatursensoren, ein dazwischen angeordnetes Heizelement und Messmittel zur Ermittlung des Heizwertes aus den Sensorsignalwerten der Temperatursensoren auf. Vorzugsweise handelt es sich um einen Durchflusssensor, wie er in der eingangs erwähnten Deutschen Patentanmeldung Nr. 199 08 664.8 und der noch unveröffentlichten Europäischen Patentanmeldung Nr. 008100511.6 beschrieben ist. Der Inhalt dieser zwei Patentanmeldungen ist Bestandteil dieser Anmeldung.

In einer ersten Variante des Verfahrens wird mittels des Heizwertsensors die Diffusivität gemessen. Obwohl der Massenfluss des Gases im Messrohr 2 aufgrund der sonischen Düse 5 konstant gehalten ist, variiert das erhaltene Sensorsignal und somit die gemessene Diffusivität mit der Zeit. Diese Änderung im Sensorsignal wird, wie bereits in der noch unveröffentlichten Europäischen Patentanmeldung Nr. 008100511.6 erkannt worden ist, von einer Änderung des kalorimetischen Wertes oder Heizwertes des durchströmenden Gases verursacht.

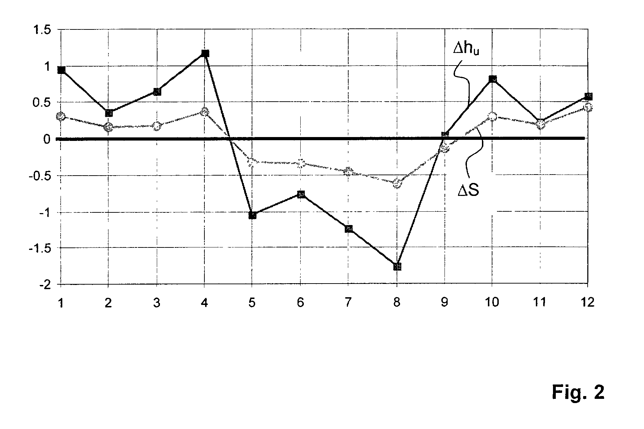

In Figur 2 ist diese Abhängigkeit dargestellt, wobei in Figur 2 eine Abweichung in Prozent von Monatsmittelwerten der Heizwerte von Erdgas während eines Jahres gezeigt ist. Diese Abweichung ist mit $\Delta h$ bezeichnet. Wie ersichtlich ist, schwankt der Heizwert um ca. 2%. Ebenfalls dargestellt und mit $\Delta S$ bezeichnet ist eine Änderung des Sensorsignalwertes $S$, wobei der Sensorsignalwert $S$ mittels des oben beschriebenen Sensors 4 erhalten worden ist. Es ist ersichtlich, dass sich der Sensorsignalwert $S$ in die gleiche Richtung ändert wie der Heizwert. Der Grund dafür, dass die Abhängigkeit nicht vollständig proportional ist, liegt darin, dass die spezifischen Heizwerte von Ethan und Propan im Verhältnis zu Methan grösser sind als die entsprechenden Signalüberhöhungen, und dass die sich ändernden Anteile von nichtbrennbaren Gaskomponenten, insbesondere von Stickstoff, keine Signaländerung hervorrufen.

Erfindungsgemäss wird nun dieser Effekt ausgenützt, indem der Durchfluss des Gasgemisches durch den Heizwertsensor 4 konstant gehalten wird und der erhaltene, sich ändernde Sensorsignalwert mit einem Kalibrier-Sensorsignalwert verglichen wird, welcher bei der Kalibration des Messgerätes von einem Kalibriergas mit bekanntem Heizwert erzeugt worden ist. Vorzugsweise wird als Kalibriergas ein Gasgemisch mit einer Zusammensetzung gewählt, das der mittleren Zusammensetzung am vorgesehenen Einsatzort entspricht. Die Differenz des Sensorsignalwertes, und somit der gemessenen Diffusivität, vom Kalibrationswert ist ein Mass für die Abweichung des zu bestimmenden Heizwertes des Messgases vom Heizwert des Kalibriergases. Im wesentlichen ist die Differenz proportional zur Änderung des Heizwertes.

Der oben beschriebene Heizwertsensor 4 ermöglicht nun je nach Art seiner Kalibration mehrere Varianten des Verfahrens:

In einer ersten Variante des erfindungsgemässen Verfahrens wird der Messgas-Massenstrom konstant gehalten, wobei dies mit der oben erwähnten sonischen Düse 5 erfolgt. Der Heizwertsensor 4 ist auf einen bestimmten, konstanten Massenstrom kalibriert, so dass das erhaltene Messsignal die Änderung der Diffusivität angibt. Der Heizwertsensor wird auf einfache Art und Weise mit einem Kalibriergas kalibriert, indem in einem ersten Schritt das Sensorsignal $S(\dot{m}, C_0)$ eines Nullgases, z. B. Stickstoff (N$_2$) bestimmt wird. In einem zweiten Schritt wird ein Sensorsignal $S(\dot{m}, C)$ für ein mittleres Gasgemisch als Referenzgas bestimmt und daraus in einem dritten Schritt ein Verstärkungsfaktor $F$ berechnet:

$$F \equiv \frac{S(\dot{m}, C_0)(h(C) - h(C_0))}{h_u(C_0)(S(\dot{m}, C) - S(\dot{m}, C_0))}$$

wobei $h(C_0)$ der Heizwert des Eichgases und $h(C)$ der Heizwert des Referenzgases ist.

Dank dieser Kalibration ist nun die relative Heizwertänderung $\Delta h(t)$ in Prozent wie folgt:

$$\Delta h(t) \equiv \frac{S(m, C(t)) - S(m, C_0)}{S(\dot{m}, C_0)} \cdot F$$

wobei $S(\cdot m, C(t))$ der zum Zeitpunkt $t$ erhaltene Sensorsignalwert des zu bestimmenden Gases ist. Das Sensorsignal ist proportional zum Diffusivität im Messgas und damit zu dessen Zusammensetzung, die den Heizwert bestimmt.

In einer zweiten Variante des Verfahrens wird zusätzlich die Dichte des Brennstoffes bestimmt, wobei hier beispielsweise der Dichtesensor 7 eingesetzt wird. Als Dichtesensor 7 eignet sich insbesondere ein Sensor mit zwei Quarzresonatoren, wie er in EP-A-0'582'045 beschrieben ist. Anhand dieser zwei gemessenen Werte, dem zur Diffusivität proportionalen Sensorsignalwert und der Dichte, wird die Genauigkeit des Systems erhöht.

In einer weiteren bevorzugten Ausführungsform der Erfindung ist der Durchflussmesser auf Energiefluss kalibriert, wobei auch hier wiederum der Massenfluss durch das Messrohr konstant gehalten wird.

Das erfindungsgemässe Verfahren und die erfindungsgemässe Vorrichtung erlauben eine einfache und kostengünstige quantitative Bestimmung einer Heizwertänderung.

**Bezugszeichenliste**

| | |
|---|---|
| 1 | Hauptleitungsrohr |
| 2 | Messrohr |
| 20 | Bypassrohr |
| 3 | Druckregulierventil |
| 4 | Heizwertsensor |
| 5 | Sonische Düse |
| 6 | Pumpe |
| 7 | Dichtesensor |
| 8 | Druckpuffer |
| 9 | Oxidationsvorrichtung |
| $\Delta h_u$ | Abweichung von Monatsmittelwerten |
| $S$ | Sensorsignal |
| $\Delta S$ | Änderung des Sensorsignals $S$ |

**Patentansprüche**

1. Verfahren zur Bestimmung einer Änderung des Heizwertes eines Gasgemisches,
**dadurch gekennzeichnet,**
**dass** die Diffusivität des Gasgemisches bei einem konstantem, vordefinierten Durchfluss gemessen wird und
**dass** die gemessene Diffusivität mit dem Referenzwert eines Kalibriergases mit bekanntem Heizwert verglichen wird, wobei eine Abweichung der gemessenen Diffusivität vom Referenzwert ein Mass für die Änderung des Heizwertes des Gasgemisches ist.

2. Verfahren nach Anspruch 1, **dadurch gekennzeichnet, dass** als Diffusivität ein Wärmestrom gemessen wird und der Massenstrom konstant gehalten wird.

3. Verfahren nach Anspruch 2, **dadurch gekennzeichnet, dass** eine Dichte des Gasgemisches gemessen wird.

4. Verfahren nach Anspruch 1, **dadurch gekennzeichnet, dass** das Eichgas und das Gasgemisch ein kohlenwasserstoffhaltiges Gasgemisch, insbesondere Erdgas, ist.

5. Vorrichtung zur Bestimmung einer Änderung des Heizwertes eines Gasgemisches mit einem Sensor zur Bestimmung einer Diffusivität,
**dadurch gekennzeichnet,**
**dass** die Vorrichtung Mittel zur Erzeugung eines konstanten Massenstroms aufweist und
**dass** die Vorrichtung mittels eines Kalibriergases mit bekanntem Heizwert auf einen Referenzwert kalibriert ist, wobei eine Abweichung der gemessenen Diffusivität vom Referenzwert ein Mass für die Änderung des Heizwertes des Gasgemisches ist.

6. Vorrichtung nach Anspruch 5, **dadurch gekennzeichnet, dass** das Mittel zur Erzeugung eines konstanten Durchflusses einen konstanten Massenfluss erzeugt, und dass die Vorrichtung auf eine Massenflussrate kalibriert ist.

7. Vorrichtung nach Anspruch 6, **dadurch gekennzeichnet, dass** sie ein Mittel zur Bestimmung der Dichte des Gasgemisches aufweist.

8. Vorrichtung nach Anspruch 5, **dadurch gekennzeichnet, dass** das Mittel zur Erzeugung eines konstanten Durchflusses einen konstanten Massenfluss erzeugt, und dass die Vorrichtung auf eine Energieflussrate kalibriert ist

9. Vorrichtung nach einem der Ansprüche 6 oder 8, **dadurch gekennzeichnet, dass** das Mittel zur Erzeugung des konstanten Massenflusses eine sonische Düse ist.

10. Vorrichtung nach Anspruch 5, **dadurch gekennzeichnet, dass** der Heizwertsensor mindestens zwei in Flussrichtung nacheinander angeordnete Temperatursensorelemente, ein dazwischen angeordnetes Heizelement und Messmittel zur Ermittlung einer Diffusivität aus Signalen der Temperatursensoren aufweist.

**Fig. 1a**

**Fig. 1b**

Fig. 2

**Europäisches Patentamt**

**EUROPÄISCHER RECHERCHENBERICHT**

Nummer der Anmeldung

EP 01 81 0546

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,Y | DE 199 08 664 A (ABB RESEARCH LTD.) 7. September 2000 (2000-09-07) * Spalte 3, Zeile 68 - Spalte 5, Zeile 65; Abbildungen 1-4 * | 1,5 | G01N33/22 G01N25/18 G01N25/20 |
| D,Y | DE 41 18 781 A (RMG MESSTECHNIK GMBH) 10. Dezember 1992 (1992-12-10) * Seite 3, Zeile 25 - Seite 4, Zeile 33; Abbildung * | 1,5 | |
| A | US 5 311 447 A (BONNE ULRICH) 10. Mai 1994 (1994-05-10) * Spalte 5, Zeile 7 - Spalte 18, Zeile 38; Abbildungen * | 1,5 | |
| A | EP 0 554 095 A (HONEYWELL INC) 4. August 1993 (1993-08-04) * Seite 5, Zeile 44 - Seite 8, Zeile 50; Abbildungen 1-7 * | 1,5 | |
| A | WO 94 10566 A (GASTEC NV ;HOERNEMANN JOHAN ADRIANUS TILM (NL)) 11. Mai 1994 (1994-05-11) * das ganze Dokument * | 1,5 | **RECHERCHIERTE SACHGEBIETE (Int.Cl.7)** G01N |
| D,A | EP 0 469 649 A (TOKYO GAS CO LTD ;OVAL ENG CO LTD (JP)) 5. Februar 1992 (1992-02-05) * das ganze Dokument * | 1,5 | |

Der vorliegende Recherchenbericht wurde für alle Patentansprüche erstellt

| Recherchenort | Abschlußdatum der Recherche | Prüfer |
|---|---|---|
| DEN HAAG | 30. November 2001 | Bosma, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**     EP 01 81 0546

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-11-2001

| Im Recherchenbericht angeführtes Patentdokument | | Datum der Veröffentlichung | Mitglied(er) der Patentfamilie | | Datum der Veröffentlichung |
|---|---|---|---|---|---|
| DE 19908664 | A | 07-09-2000 | DE | 19908664 A1 | 07-09-2000 |
| | | | WO | 0052428 A1 | 08-09-2000 |
| | | | EP | 1119744 A1 | 01-08-2001 |
| | | | NO | 20014210 A | 30-08-2001 |
| DE 4118781 | A | 10-12-1992 | DE | 4118781 A1 | 10-12-1992 |
| US 5311447 | A | 10-05-1994 | AT | 203819 T | 15-08-2001 |
| | | | AU | 3055992 A | 21-05-1993 |
| | | | CA | 2121839 A1 | 29-04-1993 |
| | | | DE | 69231977 D1 | 06-09-2001 |
| | | | EP | 0612405 A1 | 31-08-1994 |
| | | | WO | 9308457 A1 | 29-04-1993 |
| EP 0554095 | A | 04-08-1993 | EP | 0554095 A2 | 04-08-1993 |
| | | | US | 5486107 A | 23-01-1996 |
| WO 9410566 | A | 11-05-1994 | NL | 9201845 A | 16-05-1994 |
| | | | AT | 162629 T | 15-02-1998 |
| | | | AU | 672072 B2 | 19-09-1996 |
| | | | AU | 5534494 A | 24-05-1994 |
| | | | CA | 2147661 A1 | 11-05-1994 |
| | | | DE | 69316643 D1 | 26-02-1998 |
| | | | DE | 69316643 T2 | 27-08-1998 |
| | | | DK | 665953 T3 | 21-09-1998 |
| | | | EP | 0665953 A1 | 09-08-1995 |
| | | | ES | 2114164 T3 | 16-05-1998 |
| | | | FI | 951866 A | 09-06-1995 |
| | | | GR | 3026560 T3 | 31-07-1998 |
| | | | JP | 3055941 B2 | 26-06-2000 |
| | | | JP | 8505468 T | 11-06-1996 |
| | | | WO | 9410566 A1 | 11-05-1994 |
| | | | NO | 951526 A | 21-06-1995 |
| | | | RU | 2125262 C1 | 20-01-1999 |
| | | | SG | 49271 A1 | 18-05-1998 |
| | | | US | 5807749 A | 15-09-1998 |
| EP 0469649 | A | 05-02-1992 | JP | 2047928 C | 25-04-1996 |
| | | | JP | 4089538 A | 23-03-1992 |
| | | | JP | 7081918 B | 06-09-1995 |
| | | | CA | 2044197 A1 | 03-02-1992 |
| | | | DE | 69121815 D1 | 10-10-1996 |
| | | | DE | 69121815 T2 | 27-03-1997 |
| | | | EP | 0469649 A2 | 05-02-1992 |
| | | | KR | 9707816 B1 | 17-05-1997 |
| | | | US | 5167450 A | 01-12-1992 |

EPO FORM P0461

Für nähere Einzelheiten zu diesem Anhang : siehe Amtsblatt des Europäischen Patentamts, Nr.12/82